# DEMANDE DE BREVET EUROPEEN

(11) **EP 1 702 596 A2**
(43) Date de publication de la demande: **20.09.2006**
(21) Numéro de dépôt: 05301053.4
(22) Date de dépôt: 14.12.2005
(51) Int. Cl.: A61F 9/02

(54) **Masque, notamment pour la pratique des sports de glisse**

(30) Priorité: 18.03.2005 FR 0550707
(71) Demandeur: CEBE, 39400 MOREZ (FR)
(72) Inventeur: Grosjean, Nathalie, 67470, Seltz (FR)
(74) Mandataire: Vuillermoz, Bruno

(57) **Abrégé**

Ce masque comprend une monture destinée à recevoir un écran amovible (10), ladite monture étant munie de moyens (12, 13) aptes à permettre la fixation du masque sur le visage de l'utilisateur ou sur un casque porté par l'utilisateur.

L'écran (10) est muni d'organes (24, 24', 25, 25') de fixation réversible sur la monture, comprenant chacun une lumière débouchante, destinés à coopérer avec un pion de fixation (34, 34', 35, 35') de section transversale asymétrique émanant de ladite monture, et dont la rotation permet le verrouillage ou le déverrouillage de l'écran sur le masque.

## Description

### DOMAINE TECHNIQUE

L'invention concerne un masque, notamment destiné à la protection des yeux, et plus particulièrement destiné à la pratique des sports de glisse du type ski, surf..., voire de la moto et, par extension, susceptible d'être mise en oeuvre dans le cadre d'activités industrielles dès lors que la protection du visage, et plus particulièrement des yeux, est requise.

Par masque, on entend de manière traditionnelle, un dispositif protecteur muni soit d'un oculaire en une seule pièce, soit de deux oculaires séparés, conçus pour isoler les yeux de l'utilisateur, et étant apte à s'adapter au visage de ce dernier, notamment dans la partie supérieure du visage au niveau des yeux, voire éventuellement au niveau du nez.

Plus particulièrement, un tel masque est destiné d'une part, à protéger les yeux de l'utilisateur des projections, des risques mécaniques, des intempéries, et du froid ou du chaud, et d'autre part, le cas échéant, à filtrer les rayonnements de la lumière du jour, notamment la lumière solaire, lors de son utilisation.

Les masques de ce type sont le plus souvent constitués d'un écran monté sur une monture, généralement souple. Cette monture est fixée sur l'utilisateur soit par l'intermédiaire de branches, soit encore au moyen d'un serre-tête, soit encore par des pressions solidarisant le masque sur un casque.

Par ailleurs, afin de jouer son rôle protecteur, le masque est généralement maintenu plaqué contre le visage de l'utilisateur. Pour ce faire, on intercale entre la monture proprement dite et le visage, une mousse dite « de confort », par exemple en polyuréthanne, en polyéthylène ou un produit similaire, afin d'une part, de permettre une meilleure adaptabilité du masque au visage et d'autre part, d'assumer un certain amortissement des vibrations, notamment lors des efforts et sous l'effet de la vitesse. Enfin, cette mousse peut également jouer un rôle d'étanchéité, notamment vis-à-vis des intempéries.

Si les masques du type en question et connus à ce jour remplissent certes leur fonction première, l'usage démontre cependant qu'afin de permettre l'adaptabilité du masque, en principe de type standard, et donc ne comportant qu'une seule taille, à n'importe quel profil de visage des utilisateurs ou à n'importe quel type de casque sur lequel il est susceptible d'être rapporté, il importe de conférer à la monture une certaine flexibilité. Ce faisant, la qualité optique du masque s'en ressent de manière importante, notamment lorsqu'on fait subir à la monture d'importantes déformations, justement pour permettre cette adaptation. Au surplus, attendu que certains des masques proposent des écrans interchangeables, en fonction notamment des conditions météorologiques, la relative souplesse de la monture provoque quelquefois l'éjection de l'écran hors du masque, lorsque ladite monture est notamment adaptée pour de petites tailles, constituant bien évidemment un inconvénient rédhibitoire et en outre, contribuant au défaut d'étanchéité générale du masque.

### EXPOSE DE L'INVENTION

L'objet de la présente invention est donc de proposer un masque, toujours de taille standard, surmontant ces inconvénients et permettant l'interchangeabilité de l'écran constitutif de l'optique sans risquer son escamotage ou son éjection intempestifs, et optimisant en outre les conditions d'étanchéité, notamment vis-à-vis des intempéries.

Ce masque comprend une monture destinée à recevoir un écran amovible, ladite monture étant munie de moyens aptes à permettre la fixation du masque sur le visage de l'utilisateur ou sur un casque porté par l'utilisateur.

Selon l'invention l'écran est muni d'organes de fixation réversible sur la monture, comprenant chacun une lumière débouchante, destinée à coopérer avec un pion de section transversale asymétrique émanant de ladite monture, et dont la rotation par rapport à celle-ci permet le verrouillage ou le déverrouillage de l'écran sur le masque.

En d'autres termes, l'invention consiste à munir la monture d'une part, et l'écran d'autre part, de moyens de coopération, simples à mettre en oeuvre et assurant une réelle fixation de l'écran sur ladite monture, quelles que soient les déformations susceptibles d'être imposées à ladite monture pour permettre son adaptabilité au visage de l'utilisateur ou au casque que ce dernier est susceptible de porter.

Selon l'invention, les moyens de fixation du masque sur le visage ou sur le casque sont constitués d'une lanière, sangle ou équivalent, avantageusement élastique, réglable en longueur, solidarisée de manière réversible ou non au niveau de chacune de ses deux extrémités à un bras articulé sur ladite monture.

Ces bras sont articulés chacun par rapport à un axe sensiblement parallèle à l'axe de symétrie de la monture. Ils se présentent chacun sous la forme d'une ailette latérale, venant prolonger le profil général de la monture au niveau de ses parois latérales lorsqu'ils sont en position de verrouillage.

Les bras en question sont articulés sur la monture au moyen de pions traversant la paroi définissant ladite monture et assurant la fonction de pion de fixation rotatifs de l'écran mentionné précédemment. Ces pions présentent donc deux parties colinéaires, une première partie de section circulaire, propre à permettre leur rotation au sein de la monture, pourvue à cet effet d'orifices traversants, et une seconde partie à section transversale asymétrique, et plus spécifiquement, présentant une dimension plus importante que l'autre dans le plan transversal.

Selon une autre caractéristique de l'invention, l'écran est muni d'un cerclage périphérique propre à lui conférer une plus grande rigidité, les moyens de fixation réversible dont est muni l'écran émanant de ce cerclage.

En outre, selon encore une autre caractéristique de l'invention, le cerclage périphérique de l'écran comporte également des pions, notamment métalliques, destinés, outre à sécuriser le cerclage sur l'écran, également à coopérer avec des orifices prévus à cet effet au sein de la monture, notamment en zone centrale de celle-ci, de telle sorte à favoriser le placage de l'écran sur la monture, et corollairement optimiser l'étanchéité résultante du masque.

### BREVE DESCRIPTION DES DESSINS

La manière dont l'invention peut être réalisée et les avantages qui en découlent ressortiront mieux de l'exemple de réalisation qui suit, donné à titre indicatif et non limitatif à l'appui des figures annexées.

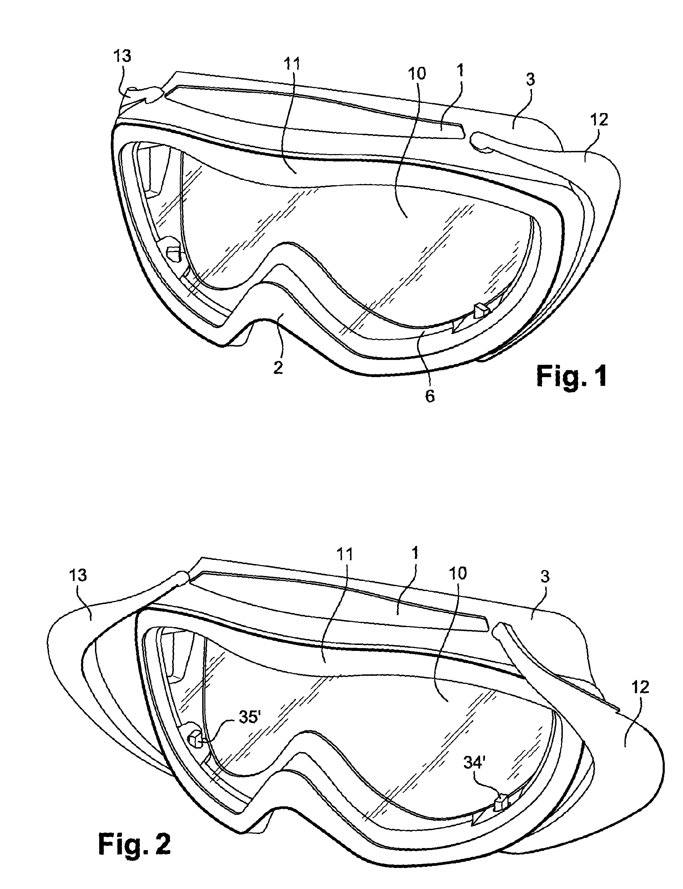

La figure 1 est une représentation schématique en perspective de la monture conforme à l'invention, l'écran étant en position verrouillée.
La figure 2 est une vue analogue à la figure 1 en position déverrouillée.

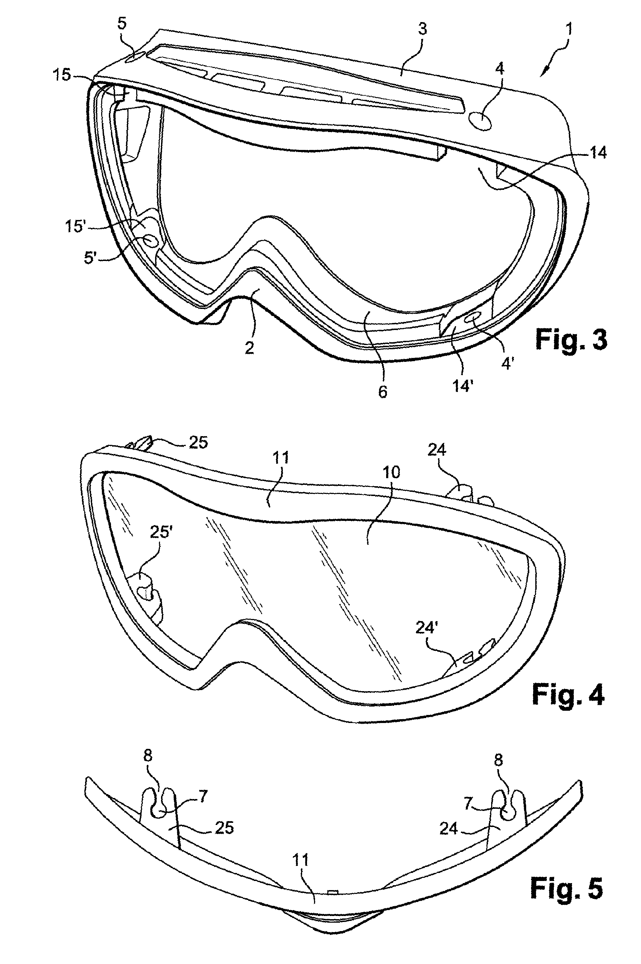

La figure 3 est une vue schématique en perspective de la seule monture.
La figure 4 est une vue schématique en perspective de l'écran mis en oeuvre conformément à la présente invention, dont la figure 5 est une vue du dessus.
La figure 6 est une vue schématique en perspective analogue à la figure 4, dans laquelle figurent les bras rotatifs de fixation réversible dudit écran sur la monture, en position verrouillée.
La figure 7 est une vue analogue à la figure 6, mais les bras étant représentés en position déverrouillée.

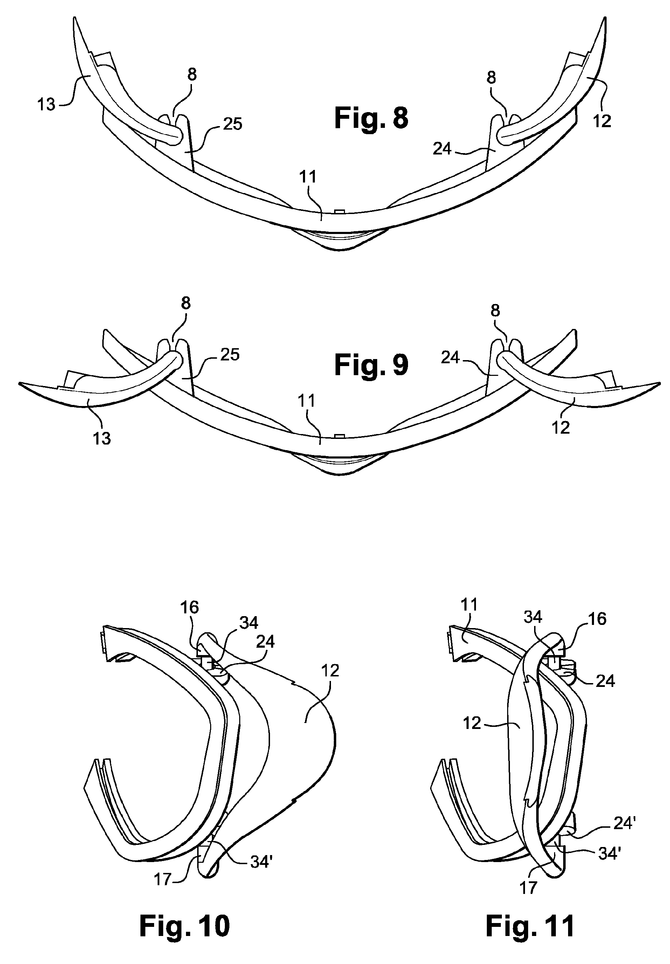

Les figures 8 et 9 sont des vues du dessus de l'écran et de ses bras de fixation, respectivement représentés en position verrouillée et déverrouillée.
Les figures 10 et 11 sont des vues latérales des figures 6 et 7, respectivement en position verrouillée et déverrouillée.
La figure 12 est une vue multiple représentant les différentes étapes de mise en place d'un écran sur la monture conforme à l'invention.

### MODE DE REALISATION DETAILLE DE L'INVENTION

Selon l'invention, le masque de protection comporte fondamentalement une monture (1) mono ou binoculaire. Cette monture est avantageusement réalisée en matière plastique par moulage, et par exemple en polyuréthanne. Elle comporte sur sa face destinée à venir prendre appui sur le visage de l'utilisateur ou sur le casque, avantageusement une lèvre (3) faisant fonction d'amortissement, susceptible en outre d'assurer une certaine étanchéité.

Cette monture (1) présente une certaine souplesse pour permettre l'adaptabilité du masque sur les différents profils des utilisateurs, voire sur différentes formes de casques. En effet et dans l'objectif évident de réduire les coûts de fabrication, une seule taille de masque est donc corollairement de monture, est donc disponible. L'adaptabilité aux différents profils et visages et aux différents types de masques ne va donc résulter que de la seule souplesse de la monture.

Celle-ci comporte une zone échancrée formant nez (2), destinée à venir prendre appui sur le nez de l'utilisateur.

Elle présente en outre un certain nombre d'orifices traversants (4, 4', 5, 5'), respectivement alignés deux à deux dans l'exemple décrit, et de section circulaire. Ces orifices traversants sont destinés à recevoir des moyens de fixation réversible (12, 13) d'un écran (10) sur ladite monture (1).

Corollairement, la face interne (6) de la paroi définissant ladite monture est pourvue de zones de dégagement (14, 14', 15, 15') formant évidements, ménagées au sein de l'épaisseur de ladite paroi, et destinées à recevoir les moyens de fixation réversible de l'écran (10), ce dernier venant obturer, de manière connue, la paroi postérieure de ladite monture.

Cet écran (10) est par exemple réalisé en polycarbonate transparent ou traité, pour éventuellement faire fonction de filtre. Selon une caractéristique de l'invention, cet écran (10) est pourvu d'un cerclage périphérique (11), destiné à lui conférer une certaine rigidité. Ce cerclage (11) est par exemple réalisé en polyamide (Grilamide LG20, marque déposée).

Le cerclage (11) comporte un certain nombre de saillies (24, 24', 25, 25'), orientées sensiblement perpendiculairement par rapport au plan moyen contenant l'écran (10), et dirigées en direction de la monture (1), lesdites saillies étant destinées à être reçues dans les évidements ou dégagements précités (14, 14', 15, 15') de la monture.

Ces saillies sont chacune munies d'une lumière (7) débouchant par une fente (8). La zone de débouchement de la fente (8) est également dirigée vers la monture. Ces saillies sont destinées à coopérer avec des pions rotatifs de fixation réversible (34, 34', 35, 35'), ménagés au sein de la monture (1), afin de permettre l'interchangeabilité de l'écran et donc son verrouillage ou son déverrouillage sur la monture.

Avantageusement, ces moyens complémentaires de fixation (34, 34', 35, 35') sont eux-mêmes associés à des moyens (12, 13) de fixation du masque sur le visage de l'utilisateur ou sur un casque, porté par l'utilisateur. Ces derniers sont constitués par deux bras, respectivement (12, 13), montés rotatifs au niveau des zones latérales extrêmes de la monture. En l'espèce, ces deux bras (12, 13), en forme d'ailettes recourbée, prolongent de manière continue ou sensiblement continue le profil de la monture lorsqu'ils sont rabattus, c'est-à-dire, lorsqu'ils sont en position de verrouillage de l'écran. Au demeurant, ces ailettes servent à fixer la sangle ou lanière ou serre-tête permettant le positionnement et la fixation du masque sur la tête de l'utilisateur ou sur le casque que ce dernier est susceptible de porter.

Ces ailettes (12, 13) sont donc articulées sur la monture (1) au moyen de pions de rotation respectivement (16, 17) pour l'ailette (12), et (18, 19) pour l'ailette (13), de section transversale circulaire, et reçus au sein des orifices traversants (4, 4', 5, 5') ménagés au sein de la monture (1). Bien évidemment, les diamètres respectifs de pions de rotation et des orifices destinés à les recevoir sont identiques au jeu près afin de permettre une rotation harmonieuse.

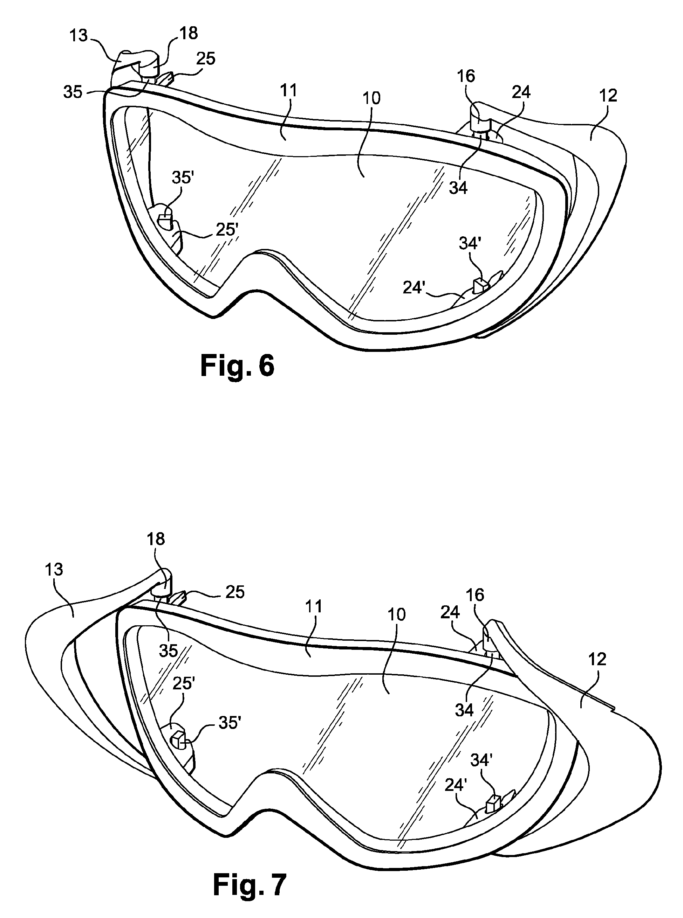

Selon l'invention, ces pions de rotation (16, 17, 18, 19) se prolongent par les pions de fixation (34, 34', 35, 35') mentionnés précédemment. Ainsi qu'on peut l'observer notamment sur les figures 6 et 7, ces pions de fixation présentent une section non circulaire, mais asymétrique, et en l'espèce, une section rectangulaire. La largeur de cette section rectangulaire correspond sensiblement à la largeur de la fente (8) de débouchement des lumières (7) des saillies (24, 24', 25, 25') émanant du cerclage (11) de l'écran (10).

Ces pions de fixation sont en outre orientés de telle sorte que, lorsque les ailettes (12, 13) sont en position de verrouillage, c'est-à-dire rabattues dans le prolongement du profil général du masque, la zone la plus étroite de ladite section, en l'espèce la largeur lorsqu'il s'agit d'une section rectangulaire, ne soit pas en regard des fentes (8) des saillies (24, 24', 25, 25').

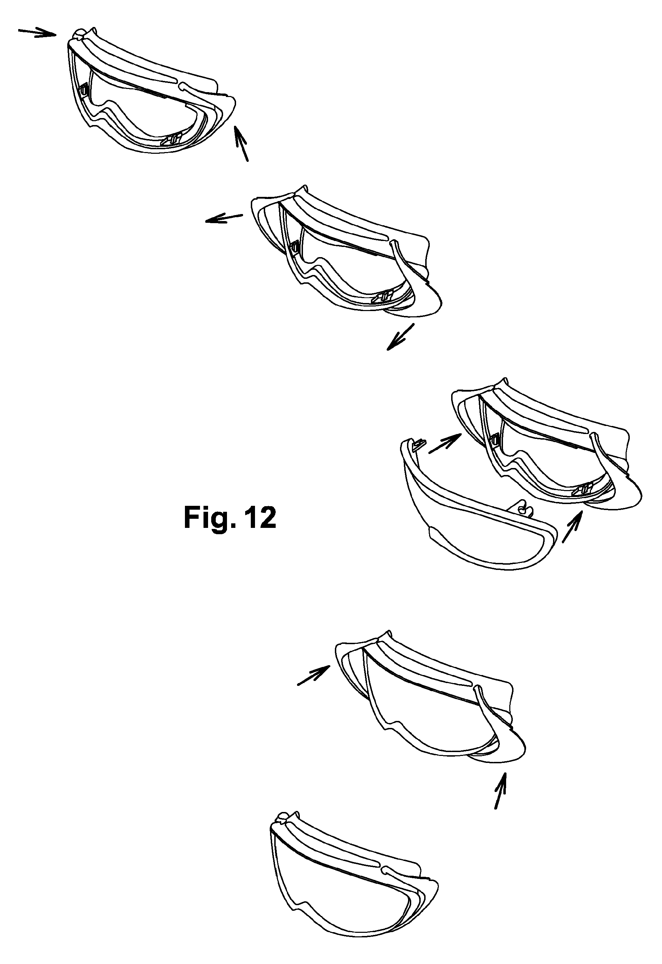

En revanche, lorsque les ailettes sont en position écartée, c'est-à-dire de déverrouillage, la conformation et le profil courbe particulier des ailettes venant en butée contre la monture, ainsi que représenté sur les figures 7 et 11, alors la largeur de la section rectangulaire vient se situer exactement ou sensiblement à l'aplomb de la fente (8) desdites saillies, permettant alors l'introduction de l'écran sur la monture ou, au contraire, son retrait lorsqu'un tel écran est déjà mis en place.

Il suffit alors, pour verrouiller l'écran remis en place, de rabattre les ailettes en direction de la monture pour ainsi assurer le verrouillage de l'écran sur la monture. Compte tenu du fait que les ailettes (12, 13) assurent également la fonction de fixation du masque sur l'utilisateur, elles sont donc tout naturellement soumises à la tension du serre-tête élastique assurant ce maintien, de sorte que par défaut, les ailettes se trouvent soumises à une contrainte assurant la position de verrouillage de l'écran sur la monture.

Avantageusement, l'ensemble constitué par une ailette, deux pions de rotation et deux pions de fixation d'un même coté, donc respectivement (12, 16, 17, 34, 34') et (13, 18, 19, 35, 35') est monobloc. Un tel ensemble est réalisé en matière plastique et est issu de moulage.

Avantageusement, le cerclage (11) de l'écran (10) comporte en outre des pions, notamment métalliques (non représentés), s'étendant également selon une direction sensiblement perpendiculaire au plan moyen contenant l'écran.

Ces pions métalliques sont destinés tout d'abord à coopérer avec des orifices prévus à cet effet, ménagés au sein de la face en regard de la paroi définissant la monture, et plus particulièrement situés en zone centrale, c'est-à-dire au voisinage du nez (2). Ce faisant, cela favorise le placage de l'écran sur la monture, et corollairement optimise l'étanchéité résultante du masque, puisque aussi bien, il n'y a alors plus de risque de « bâillement » de l'écran à ce niveau.

Au surplus, la mise en oeuvre de ces pions métalliques supplémentaires permet également de sécuriser la solidarisation du cerclage (11) sur la périphérie de l'écran (10).

On a schématisé au sein de la figure 12 les différentes étapes de fixation d'un écran sur la monture du masque de l'invention.

Succinctement, il convient en premier lieu d'écarter les deux ailettes (12, 13) et les venir placer en butée contre la monture, de telle sorte à placer ladite monture en position déverrouillée.

Puis, on introduit les saillies (24, 24', 25, 25') de l'écran dans les évidements correspondants (14, 14', 15, 15') de la monture, l'orientation des pions de fixation étant telle que cette introduction est rendue possible, et le cas échéant les pions métalliques dans les orifices prévus à cet effet au sein de ladite monture.

On rabat alors les ailettes en direction du plan général de la monture, de telle sorte à verrouiller l'écran sur ladite monture.

On conçoit ainsi l'extrême simplicité des opérations d'interchangeabilité de l'écran pour un masque conforme à l'invention. En outre et surtout, de par le mode de fixation mis en oeuvre et notamment le verrouillage de l'écran au niveau de la monture, et plus particulièrement au niveau des cotés de celle-ci, il n'y a pas risque de voir l'écran être éjecté de la monture lorsque ladite monture est courbée pour permettre l'adaptation du masque sur un visage ou sur un casque particulier.

En outre, la mise en oeuvre du cerclage particulier au niveau de l'écran permet d'optimiser l'étanchéité du masque, que celui-ci soit mis en place directement sur le visage de l'utilisateur ou sur un casque.

## Revendications

1. Masque comprenant une monture (1) destinée à recevoir un écran amovible (10), ladite monture étant munie de moyens (12, 13) aptes à permettre la fixation du masque sur le visage de l'utilisateur ou sur un casque porté par l'utilisateur, ***caractérisé* en ce que** l'écran (10) est muni d'organes (24, 24', 25, 25') de fixation réversible sur la monture (1), comprenant chacun une lumière débouchante (8), destinés à coopérer avec un pion de fixation (34, 34', 35, 35') de section transversale asymétrique émanant de ladite monture, et dont la rotation permet le verrouillage ou le déverrouillage de l'écran sur le masque.

2. Masque selon la revendication 1, ***caractérisé* en ce que** les moyens de fixation du masque sur le visage ou sur le casque sont constitués d'une lanière, sangle ou équivalent, avantageusement élastique, réglable en longueur, solidarisée de manière réversible ou non au niveau de chacune de ses deux extrémités sur un bras (12, 13) articulé sur ladite monture (1).

3. Masque selon la revendication 2, ***caractérisé* en ce que** les bras (12, 13) sont articulés sur la monture (1), chacun par rapport à un axe sensiblement parallèle à l'axe de symétrie de la monture, et **en ce qu'**ils se présentent chacun sous la forme d'une ailette latérale, venant prolonger le profil général de la monture au niveau de ses parois latérales lorsqu'ils sont en position de verrouillage.

4. Masque selon l'une des revendications 2 et 3, ***caractérisé* en ce que** les bras (12, 13) sont articulés sur la monture (1) au moyen de pions de rotation (16, 17, 18, 19) traversant la paroi définissant ladite monture au niveau d'orifices traversants (4, 4', 5, 5'), lesdits pions de rotation se prolongeant chacun par un pion de fixation (34, 34', 35, 35') de l'écran (10).

5. Masque selon la revendication 4, ***caractérisé* en ce que** les pions issus des bras (12, 13) présentent deux parties colinéaires, une première partie de section circulaire, propre à permettre leur rotation au sein des orifices traversants (4, 4', 5, 5') de la monture (1), et faisant fonction de pions de rotation, et une seconde partie à section transversale asymétrique, et plus spécifiquement, présentant une dimension plus importante que l'autre dans le plan transversal, destinée à coopérer avec les d'organes (24, 24', 25, 25') de fixation réversible de l'écran (10) sur la monture (1), afin de faire fonction de pion de fixation.

6. Masque selon l'une des revendications 1 à 5, ***caractérisé* en ce que** les organes (24, 24', 25, 25') de fixation réversible de l'écran (10) sur la monture (1) comportent chacun une lumière (7) débouchant par une fente (8) en direction de la monture avec laquelle ils sont destinés à coopérer, la fente étant de largeur inférieure à la plus grande dimension de la section transversale des pions de fixation (34,34',35,35').

7. Masque selon l'une des revendications 1 à 6, ***caractérisé* en ce que** l'écran (10) est muni d'un cerclage périphérique (11), destiné à lui conférer une plus grande rigidité.

8. Masque selon la revendication 7, ***caractérisé* en ce que** les moyens de fixation réversible (24, 24', 25, 25') dont est muni l'écran (10) émanent du cerclage (11).

9. Masque selon la revendication 7, ***caractérisé* en ce que** le cerclage périphérique (11) de l'écran (10) comporte également des pions, notamment métalliques, destinés, outre à sécuriser le cerclage sur l'écran, également à coopérer avec des orifices prévus à cet effet au sein de la monture, notamment en zone centrale de celle-ci, de telle sorte à favoriser le placage de l'écran sur la monture, et corollairement à optimiser l'étanchéité résultante du masque.
